(19) Europäisches Patentamt
European Patent Office
Office européen des brevets

(11) **EP 2 050 481 B1**

(12) **EUROPÄISCHE PATENTSCHRIFT**

(45) Veröffentlichungstag und Bekanntmachung des Hinweises auf die Patenterteilung:
**15.06.2011 Patentblatt 2011/24**

(51) Int Cl.:
***A61N 2/02*** *(2006.01)*

(21) Anmeldenummer: **07020322.9**

(22) Anmeldetag: **17.10.2007**

(54) **Vorrichtung zur Magnetfeldtherapie**

Device for magnetic field therapy

Dispositif de thérapie de champ magnétique

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**22.04.2009 Patentblatt 2009/17**

(73) Patentinhaber: **Kafka, Wolf A., Prof. Dr.**
**82288 Kottgeisering (DE)**

(72) Erfinder: **Kafka, Wolf A., Prof. Dr.**
**82288 Kottgeisering (DE)**

(74) Vertreter: **Kafka, Veit-Stefan**
**Vorhoelzerstrasse 21**
**81477 München (DE)**

(56) Entgegenhaltungen:
**EP-B1- 0 594 655      EP-B1- 0 995 463**
**DE-A1- 10 313 259**

**Beschreibung**

Technisches Gebiet der Erfindung

Die Erfindung betrifft eine Vorrichtung zur Magnetfeldtherapie, wie sie z.B. in EP 0 995 463 B1 beschrieben ist.

Stand der Technik

Seit Beginn der 70iger Jahre sind Vorrichtungen bekannt, die zum Teil auch routinemässig vornehmlich im Bereich der Orthopädie in Kliniken, ärztlichen Praxen und in der Heimanwendung zu therapeutischen Zwecken eingesetzt werden.

Ein Generator dient dabei zur Ansteuerung einer Magnetfelderzeugungsvorrichtung, wobei der Generator die Magnetfelderzeugungsvorrichtung so ansteuert, dass das Magnetfeld aus einer Vielzahl von, hinsichtlich zeitlichem Abstand und Amplitudenverlauf charakteristisch geformten Grundimpulsen bzw. Hauptimpulsen besteht, deren Impulshäufigkeit üblicherweise zwischen 0 und 1000 Hz liegt. Ein derartiger Hauptimpuls kann z.B. durch sinus-, trapez-, sägezahnförmig (EP 0 594 655 B1 (König Herbert), EP 0729 318 B1 (Fischer Gerhard, EP A 0 377 284) oder wie in EP 0 995 463 B1 (Kafka Wolf A) durch einen im Mittel exponentiell ansteigenden sinusförmig modulierten Feldintensitätsverlauf mit magnetischen Flussdichten in Bereichen von nanoTesla bis mehreren milliTesla realisiert werden, derart dass sich die Hauptimpulse, wie beispielsweise in EP 0 995 463 B1, aus einer Reihe zeitlich aufeinanderfolgender Unterimpulse zusammensetzen, die sich in ihren Amplituden und/oder Anstiegs- bzw. Abfall-Steilheiten, letztlich somit auch in ihrer individuellen Dauer unterscheiden.

Die Erzeugung der magnetischen Felder erfolgt häufig durch ein oder mehrere von einander auch unabhängig angesteuerten elektrischen Spulen. Aus der Vielzahl von Vorrichtungen zur elektromagnetischen Therapie finden sich entsprechend auf die Generation zeitveränderlicher Feldintensitäten ausgerichtete Dokumente beispielsweise in EP 1 364 679 A2, EP-A-0 266 807, EP-A-0 266 907, DE-A-4 221 739, US-A-5 181 902, WO-A-96/32159, UA-A-4 428 366, EP 0 995 463 B1.

Die therapeutische Applikation erfolgt aus Gründen des operativen Aufwands und der damit verbundenen Risiken heute üblicherweise nichtinvasiv.

Die Beeinflussung des biologischen Systems beruht nach gängiger Vorstellung auf einem in seinen Energieanteilen noch unbekannten Zusammenwirken der durch die Vorrichtungen generierten magnetischen und elektrischen Feldanteile. Die ausgelösten physiologischen und biologischen Wirkungen basieren danach auf der direkten, durch magnetische oder/und nach dem Prinzip der Induktion (Maxwellschen Gleichungen) der indirekten, durch elektrische Kraftwirkungen verursachten Beeinflussung (energetische Aktivierung) der physikalischen-chemischen Reaktivität der an den naturgegebenen und auf Selbsterhalt ausgerichteten Regulationsmechanismen beteiligten ionalen, atomaren und molekularen, im Folgenden pauschal als Molekülstrukturen bezeichneten Bausteinen.

So wird beispielsweise beschrieben, dass der Einsatz der speziellen Vorrichtung EP 0 995 463 B1 gegenüber unbehandelten biologischen Objekten als Kontrollen zu einer Reihe differenzierter physikalisch-physiologisch Prozesse führt wie z.B. zur signifikanten Aktivierung von Prozessen:

- bei der Bildung energiereicher Verbindungen insbesondere von Adenosintriphosphat (ATP) und Bis-2-3-phospoglycerat (BPG) in humanen Erythrozyten [Spodaryk K (2001) Red blood metabolism and haemoglobin oxygen affinity: effect of electromagnetic fields on healthy adults. In: Kafka WA (ed) 2nd Int World Congress Bio-Electro-Magnetic-Energy-Regulation. Emphyspace 2: 15-19; Kafka WA, Spodaryk K (2003) Effects of extremely weak BEMER 3000 type pulsed electromagnetic fields on red blood metabolism and hemoglobin oxygen affinity. Fizoterapia 11 (3): 24-31]

- zur Verbesserung des Funktionszustands der Mikrozirkulation insbesondere hinsichtlich des Durchblutungsverhaltens (besonders auch bei Diabetes-bedingten Durchblutungsstörungen) und der Sauerstoffutilisation [Klopp R (2004) Vitalmikroskopische und reflexionsspektrometrische Untersuchungen zur Wirkung des Gerätesystems "BEMER 3000" auf den Funktionszustand der Mikrozirkulation. Bericht aus dem Institut für Mikrozirkulation, Berlin; Klopp R, Niemer W (2007) Einfluss eines pulsierenden elektromagnetischen Feldes mit vasomotorischer Stimulation auf einen eingeschränkten Funktionszustand der Mikrozirkulation. Komplement. Integr. Med 08/2007: 47-53] sowie

- im Ablauf von Schutzmechanismen insbesondere hinsichtlich eines in Klopp 2004 beschriebenen beschleunigten Ablaufs infektiös ausgelöster, durch komplexes Zusammenspiel von Signal- und Adhäsionsmolekülen getragener Leukozyten-Immunabwehrreaktionen

- bei der Protektion gegen chemische Stressfaktoren insbesondere der Reduktion chemisch (durch das Teratogen Cyclophosphamid) induzierter Missbildungen in der Ontogenese von warmblütigen Wirbeltierembryonen (am Model von Hühnereiern) [Jelínek R, Bláha J, Dbalý Jaroslav (2002) The electromagnetic BEMER 3000 signal modifies response to teratogens. In: Kafka WA (ed) 3nd Int. World Congress Bio-Electro-Magnetic Energy-Regulation, Bad-Windsheim, Germany, Emphyspace 3

- bei Reparationsvorgängen insbesondere hinsichtlich einer verbesserten Heilung standardmässig erzeugter Wunden [Kafka WA, Preißinger M (2002) Verbesserte Wundheilung durch gekoppelte, BEMER 3000 typisch gepulste, Elektromagnetfeld- und LED-Licht-Therapie am Beispiel vergleichender Untersuchungen an standardisierten Wunden nach Ovariektomie bei Katzen (felidae). In: Edwin Ganster (Hrsg) Österreichische Gesellschaft der Tierärzte (ÖGT) Kleintiertage-Dermatologie 2.-3. März 2002, Salzburg Congress]

- bei anti-oxidativen Regulationen insbesondere hinsichtlich enzymatisch und spektralphotometrisch bestimmter beschleunigter Reduktionsumsatzraten [Spodaryk K (2002) The effect of extremely weak electromagnetic field treatments upon signs and symptoms of delayed onset of muscle soreness: A placebo controlled clinical double blind study. Medicina Sportiva 6: 19-25; Klopp R, Niemer W, Pomrenke P, Schulz J (2005) Magnetfeldtherapie: Komplementär-therapeutisch sinnvoll oder Unsinn? Stellungnahme unter Berücksichtigung neuer Forschungsergebnisse mit dem Gerätesystem BEMER 3000, Institut für Mikrozirkulation, Berlin]

- bei der Leistungsteigerung im Spitzensport [Spodaryk K and Kafka WA (2004) The influence of extremely weak BEMER3000 typed pulsed electromagnetic fields on ratings of perceived exertion at ventilatory threshold. In: Marincek C, Burger H (eds) Rehabilitation Sciences in the New Millennium Challenge for Multidisciplinary Research. 8th Congress of EFRR, Ljubljana. Medimont International Proceedings: 279-283]

- von Replikations- und Proliferationsmechanismen insbesondere hinsichtlich einer signifikanten Reduktion des Tumorwachstums in thymusfreien nicht aber in vergleichend untersuchten normalen Mäusen [Rihova B (2004) Die Wirkung der elektromagnetischen Felder des BEMER 3000 auf das Wachstum des experimentellen Mäuse-EL 4T Zellen-Lymphoms, SAMET Kongress, Interlaken; Rihova B, Dbaly J, Kafka WA Exposure to special (BEMER-type) pulsed weak electromagnetic fields does not accelerate the growth of mouse EL4 T cell lymphoma, submitted]

- der Proteinbildung und -Aktivierung insbesondere hinsichtlich differentieller up- und down- Regulation genexprimierter Proteinmengen. Im Rahmen einer Genchip-Analyse konnte gezeigt werden, dass die Anwendung der Vorrichtung EP 0 995 463 B1 auf Stammzellen des Knochenmarks (Knochen und Knorpelzellen) gegenüber nichtbehandelten die Menge der produzierten Proteine unterschiedlich beeinflusst: die Menge (Expression) der produzierten Proteine ist also teils erhöht, teils erniedrigt, teils bleibt sie (interessanterweise unter anderem hinsichtlich der Expression von Onkogenen) unbeeinflusst [Kafka WA, Schütze N, Walther M (2005) Einsatz extrem niederfrequent (BEMER typisch) gepulster schwacher elektromagnetischer Felder im Bereich der Orthopädie (Application of extreme low frequent (BEMER type) pulsed electromagnetic fields in orthopedics). Orthopädische Praxis 41 (1): 22-24; Walther M, Meyer F, Kafka WA, Schütze N (2007) Effects of weak, low frequency pulsed electromagnetic fields (BEMER type) on gene expression of human mesenchymal stem cells and chondrocytes: an in vitro study. Electromagnetic Biology and Medicine, Manuscript ID: 257936].

- psychovegetativer Prozesse insbesondere der Reduktion der (Zahnarzt-) Angst durch eine der Zahnbehandlung unmittelbar vorrausgehende elektromagnetische lokale Stimulation des Solarplexus [Michels-Wakili S and Kafka WA (2003) BEMER 3000 pulsed low-energy electromagnetic fields reduce dental anxiety: a randomized placebo controlled single-blind study. 10th International Congress on Modern Pain Control 5-8 June 2003 Edinburgh, GB]

- der Reduktion lumbargisch initiierter Folgereaktionen insbesondere der Reduktion von Bewegungsschmerz, Schlaflosigkeit und Angst [Bernatzky G, Kullich W, Aglas F, Ausserwinkler M, Likar R, Pipam W, H. Schwann H, Kafka WA (2007) Auswirkungen von speziellen, (BEMER-typisch) gepulsten elektro-magnetischen Feldern auf Schlafqualität und chronischen Kreuzschmerz des Stütz- und Bewegungsapparates (low back pain): Eine doppelblinde randomisierte Duo Center Studie (Der Schmerz, in press].

- der analgetischen Wirkung, insbesondere hinsichtlich der Reduktion polyneuropathischer Schmerzzustände als Folge von oxidativem Stress nach Chemotherapie [Gabrys M (2004) Pulsierende Magnetfeldtherapie bei zytostatisch bedingter Polyneuropathie. Deutsche Zeitschrift für Onkologie 36: 154-156].

Weitere zusammenfassende Berichte finden sich bei: Carpenter DO, Aryapetyan S (1994) Biological effects of electric and magnetic elds: sources and mechanism, vol 1. Beneficial and harmful effects, Vol 2. Academic Press; Bohn W, Kafka WA (2004) Energie und Gesundheit: BEMER 3000 Bio-Elektro-Magnetische-Energie-Regulation nach Prof. Dr. Wolf A. Kafka. Haug Verlag, Stuttgart (Thieme Verlagsgruppe): 1-130; Kafka WA (2006) The BEMER 3000 Therapy: A new complementary "electro-magnetic drug" effectively supports widespread scattered prophylactic and therapeutic treatments. In: Kochueva E (ed) Achievements in space medicine into health care practice and industry 3rd European praxis matured congress KOPIE-DRUCK sponsored by ESA, DLR & POCKO MOC]; Quittan M, Schuhfried O, Wiesinger GF, Fialka-Moser V (2000) Klinische Wirksamkeiten der Magnetfeldtherapie - eine Literaturübersicht. Acta Medica Austriaca 3:61-68; Matthes Rudiger (2003) Guidance on determining compliances of exposure to pulsed and complex non sinusoidal waveforms below 100 khz with ICNIRP GUIDELINES. The International Commission on Non-Ionizing Radiation Protection ICNIRP Secretariat, Bundesamt für Strahlenschutz, Institut für Strahlenhygiene, Ingolstädter Landstrasse, D-85764 Oberschleissheim, Germany].

Diese Ergebnisse, insbesondere diejenigen zur differenzierten Wirkung auf das Tumorwachstum und die Genexpression, bestätigen und implizieren die einleitend aufgeführte Annahme, dass die der elektromagnetisch induzierten biologischen Wirkungen auf der Aktivierung ursächlich unterschiedlicher molekularen Mechanismen beruhen, und sich, insbesondere bezogen auf die beschriebene differenzierte Genexpression z.B. nicht mit der Verbesserung des Zustands der Mikrozirkulation erklären lassen. Entsprechend der demzufolge zur jeweiligen Aktivierung benötigten unterschiedlichen Energiemenge bestätigt sich damit die (bereits in den Maxwellschen Gleichungen beschriebene) Bedeutung, die der (durch unterschiedliche Amplituden und Flankensteilheiten der überlagerten Unterimpulse) gekennzeichneten Zeitintensitätsverteilung zukommt. - Entsprechend der auf diese Weise differenzierten "elektromagnetischen" Wirkstoff-Eigenschaften kommt den zeitlichen Feldintensitätsvariationen in der Elektromagnetfeldtherapie somit eine ähnliche Bedeutung zu wie den Struktur-Aktivitätsbeziehungen von Arzneimittelwirkstoffen in der Pharmazie.

Nur am Rande sei auf Berichte der WHO hingewiesen, demzufolge der Einsatz derartiger schwachenergetischen Vorrichtungen keine schädlichen Nebenwirkungen erwarten lässt [Electromagnetic Fields (EMF) ff. *http://www.who.int/peh-emf/en/; http://www.who.int/topics/electromagnetic_fields/en/*]. In diese Richtung weist auch ein Bericht einer für die Zertifizierung von Medizinprodukten zuständigen deutschen Aufsichtsbehörde [LGA-Bericht 2005], der speziell für die - nach statistischen Erhebungen seit 1998 bis heute abgeschätzt mehrere millionenfach eingesetzte - Vorrichtung EP 0 995 463 B1 keine gesundheitlich negativen Auswirkungen bestätigt.

Zugrundeliegende Problematik

Alle bisher bekannten Vorrichtungen zur Behandlung des menschlichen Körpers führen zwar zu der erwünschten beschleunigenden Wirkung von Heilungsvorgängen, lassen sich aber in ihrer Wirkung noch verbessern. Ohne Berücksichtigung dessen ob ein Patient tatsächlich von einer derartigen Behandlung profitiert, beziehen sich die Aussagen darüber hinaus in Form sogenannter Surrogatparameterstudien vielfach nur auf die Behandlung von Laborwerten wie z.B. zu Parametern des Kreislaufs, des Fetttransports (LDL, HDL), der Lungenfunktion, der Knochendichte oder sonstiger Stoffumsätze. Das ist insofern problematisch, als zur Erzielung eines deutlich beschleunigten Heilerfolges die Anwendung häufig, oft auch unter Variation der Feldintensitätsverhältnisse, wiederholt werden muss, was nicht nur zu einer erhöhten Belastung der Patienten, sondern auch zu deutlich höheren Behandlungskosten führt.

Offensichtlich ist der Zusammenhang zwischen den zeitlichen Intensitätsverhältnissen der applizierten elektromagnetischen Felder und der jeweils induzierten biologischen Wirkung in der Vorrichtung EP 0 995 463 B1 noch unzureichend ausgeschöpft. Gemäss der erwähnten Vorstellungen zur Wirkungen verfügen dort insbesondere die in ihrer absoluten (unabhängig vom Vorzeichen) Intensität gegenüber den darauf folgenden steiler und höher ansteigenden Unterimpulse des Hauptimpulses auf Grund ihrer Amplituden- und Flanken- Steilheiten über ein prinzipiell geringeres Potenzial zur individuellen Beeinflussung der die physiologischbiologischen Reaktionen modulierenden Aktivierungsenergien. Einfach ausgedrückt, die in EP 0 995 463 B1 generierten zeitlich unmittelbar aufeinander Unterimpulse eines Hauptimpulses sind hinsichtlich ihrer Amplitude und Steilheit stets kleiner als die darauf folgenden. Dementsprechend und zusätzlich wegen der sich gemäss der Maxwellschen Gleichungen somit unterschiedlich aufbauenden elektrischen Feldkomponenten liefern diese gegenüber den jeweils nachfolgenden Unterimpulsen im Sinne der angestrebten möglichst breit gestreuten Aktivierung von Regulationsmechanismen nur einen dementsprechend eingeschränkten geringeren energetischen Beitrag. Unter Bezugnahme auf die in EP 0 995 463 B1 beschriebenen Hüllkurven als Verbindung der oberen bzw. der unteren Extremwerte der Unteramplituden, kommt hinzu, dass sich die der Koordinatenachse näherliegende Hüllkurve von dieser innerhalb des Impulsablaufs zunehmend entfernt: Gemessen an der Amplitude würden also aufeinanderfolgende Unterimpulse gegenüber solchen, bei denen sich die beschriebene Hüllkurve von der Koordinatenachse weniger "entfernt" über ein geringeres Aktivierungspotential verfügen. - Im Sinne der angestrebten breiten Aktivierung sind demnach die in EP 0 995 463 B1 beschriebenen Formen der Haupt- und Unterimpulse hinsichtlich Dauer und Amplitude und Amplitudenabfolge noch unzureichend abgestuft.

Aufgabe und Problemlösung

Aufgabe der Erfindung ist es, eine Vorrichtung zur Magnetfeldtherapie und ein magnetisches Signal anzugeben, das sich, insbesondere bezogen auf Amplituden, Flankensteilheiten und Abfolge der einzelnen Unterimpulse, durch eine feinere Abstufung und damit eine breitere spektrale Zusammensetzung auszeichnet und somit, ein breites Band an elektromagnetisch aktivierbaren Molekularstrukturen ansprechend, eine breitere physiologische Wirkungsbreite gewährleistet. Die Erfindung ist damit ausgerichtet auf eine möglichst breitwirksame energetische Unterstützung der den normalen Lebensablauf bestimmenden komplex vernetzten molekularen Regulationsprozesse. Abweichend von den üblicherweise Symptom orientierten Behandlungsweisen verfolgt sie dementsprechend ein ganzheitliches und somit präventives, gleichwie auf Regeneration, Erhalten und Wohlbefinden ausgerichtetes Therapiekonzept.

Diese Aufgabe wird erfindungsgemäss durch die unabhängigen Patentansprüche gelöst, wobei vorteilhafte Weiterbildungen in Unteransprüchen angegeben sind.

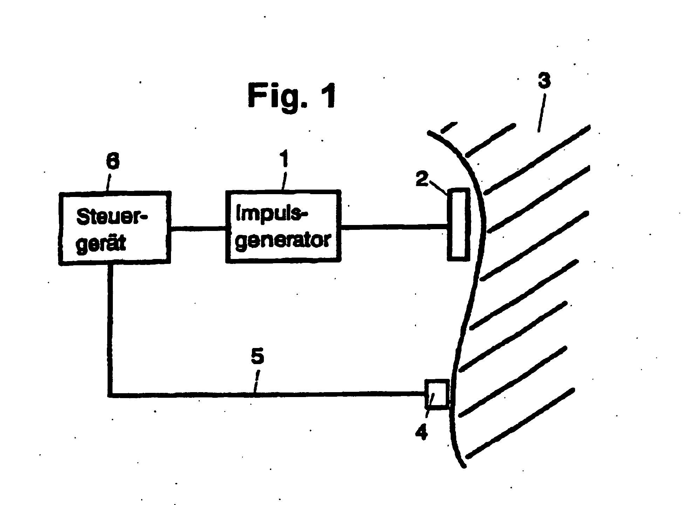

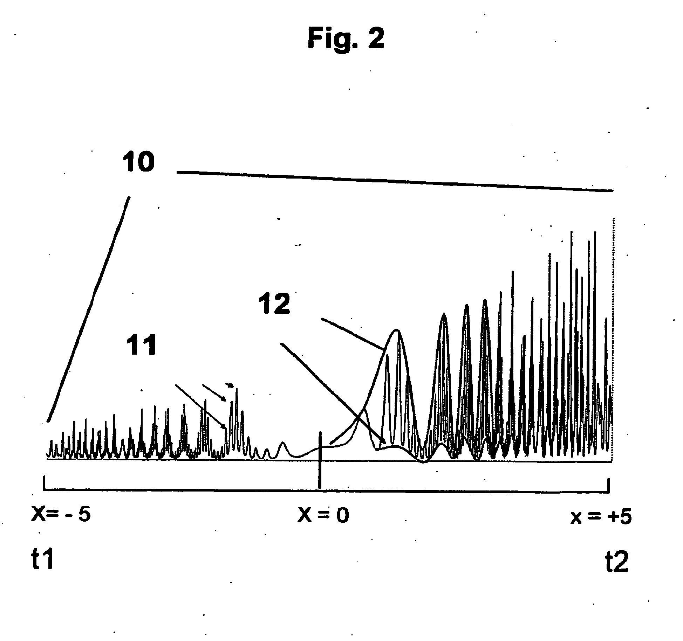

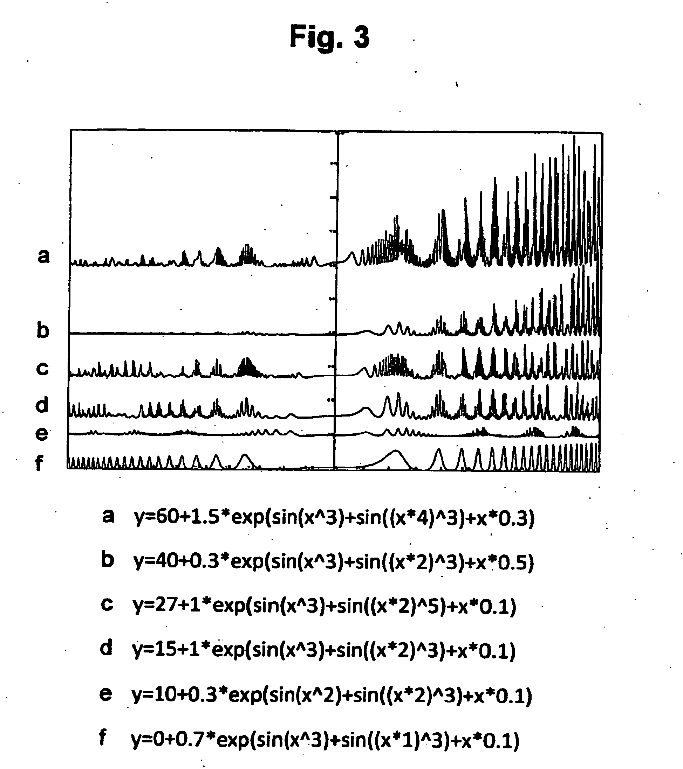

Damit wird eine Vorrichtung geschaffen, die einen Impulsgenerator und eine Felderzeugungsvorrichtung zur Erzeugung eines elektromagnetischen Feldes umfasst (Fig. 1). Der Impulsgenerator dient zur Ansteuerung der Felderzeugungsvorrichtung, wobei der Impulsgenerator die Felderzeugungsvorrichtung über geeignete Strom- oder Spannungsabläufe so ansteuert, dass sich das pulsierende magnetische Feld aus einer Reihe von individuell einstellbaren Hauptimpulsen und einer Vielzahl von darin hinsichtlich Flankensteilheiten und Amplituden feiner abgestuften Unterimpulsen derart zusammensetzt, dass die spektrale Zusammensetzung eine möglichst grosse Energiedichte erreicht. Ein derartiger Hauptimpuls kann sich dabei aus einer hinsichtlich ihrer Amplitude im Mittel gleichbleibenden bzw. nach Art einer Potenzfunktion im Mittel an- oder absteigenden Amplitude Abfolge von in ihren Flankensteilheiten unterschiedlichen Unterimpulsen zusammensetzen. Charakterisiert durch die Verbindungslinen der Extrema (Hüllkurven) der einzelnen Unterimpulse können die Hauptimpulse, wie im Folgenden und unter Fig. 2 und Fig. 3 genauer ausgeführt, abhängig von den gewählten Bedingungen selbst einen pulsförmigen Verlauf annehmen. Insbesondere kann erreicht werden, dass der mittlere Abstand zwischen der Koordinatenachse und einer der dieser zugewandten Hüllkurve innerhalb eines Hauptimpulses, abweichend von EP 0 995 463 B1, weitgehend konstant bleibt.

Bei einer Spannungsansteuerung ist darauf zu achten, dass die Induktivität der Spule dazu führt, dass das applizierte Spannungssignal zu keinem identischen Magnetfeldsignal führt, weil die Induktivität der Spule den Signalverlauf zu höheren Frequenzen hin mit geringeren Amplituden wiedergibt. Dem kann leicht dadurch entgegengewirkt werden, dass die gewünschte zeitliche Funktion des Magnetfeldes über eine Fouriertransformation in den Frequenzbereich überführt, dort die hohen Frequenzen entsprechend angehoben werden, um das dann geeignete Zeitsignal für den Spannungsverlauf zu erhalten. Eine andere Möglichkeit besteht darin, einen spannungsgesteuerten Stromverstärker zu verwenden, der der Spule Stromsignale mit einem Verlauf appliziert, der dem gewünschten Magnetsignal entspricht.

Die Behandlungsdauer ergibt sich aus der Summe der Zeitabschnitte für die Applikation der einzelnen Hauptimpulse und der dazwischenliegenden Pausen /ta - tb/. Für den jeweils zwischen 2 Erholungspausen liegenden Amplitudenverlaufs y = f(t) ergibt sich im Zeitbereich t1 < t < t2 eines Hauptimpulses mit

$$y = f(t) \text{ und } t = f(x) \text{ und } t = (t2 - t1)^* x/(x2 - x1) \text{ mit } x1 < x < x2.$$

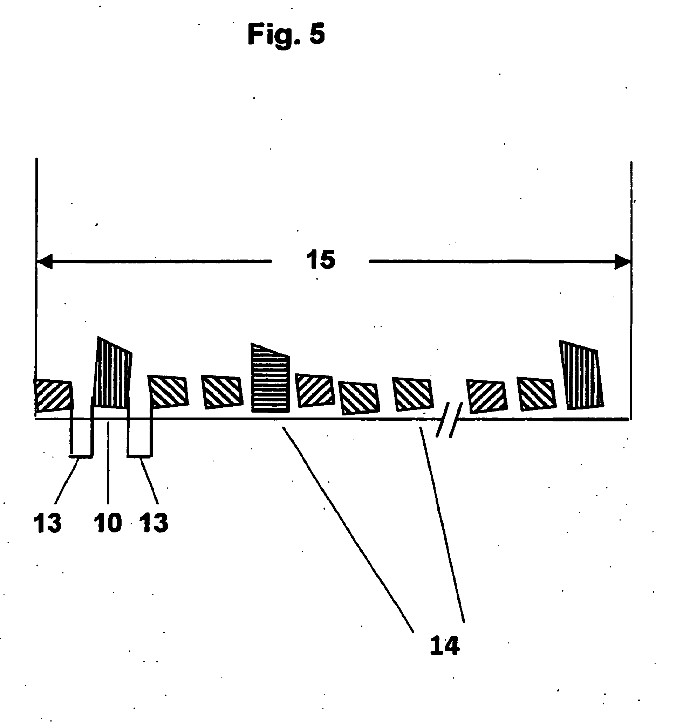

erfindungsgemäss ein Amplitudenverlauf mit der folgenden Zeitfunktion (Fig. 2 bis Fig. 5):

$$y(x) = k1 + k2^* EXP(SIN(x^{\wedge}k3) + SIN((x^*k4)^{\wedge}k5) + x^*k6);$$

Die Funktion beschreibt einen im wesentlichen durch einen durch die Parameter k1, k2, k3, k4, k5 und k6 definierten exponentiellen Modulationsverlauf, wobei diesen Modulationsverlauf zwei SIN-Funktionen beherrschen, die durch die Parameter k3, k4 und k5 bestimmt sind. Nachdem eine SIN-Funktion nur Werte zwischen +1 und -1 annehmen kann, liegt die Summe der beiden die EXP-Funktion bestimmenden SIN-Funktionen zwischen -2 und +2. Die EXP-Funktion kann deshalb nur Werte zwischen EXP(-2) = 0,135 und EXP(+2) = 7,39 annehmen, wenn man von dem weiteren Term +x*k6 absieht, der dann einen weiteren Anstieg festlegt. Die SIN-Funktionen selbst mit den zugehörigen Parametern sind derart gewählt, dass eine Feinabstufung der aufeinanderfolgenden Amplitudenwerte in den damit definierten Unterimpulsen mit hoher Variationsbreite erreicht wird.

Darin bedeuten:

    o y(x) = Amplitude des Magnetfeldes innerhalb eines Hauptimpulses als Funktion von x;
    o x = rechnerische Ersatzgrösse für die Zeit t (siehe unten);
    o k1 = Zahlenwert;

o k2 = Multiplikationsfaktor für die Funktion EXP(SIN(x^k3)+SIN((x*k4)^k5)+x*k6);

o k3 = Exponent von x;

o k4 = Multiplikationsfaktor von x;

o k5 = Exponent von (x*k4);

o k6 = Multiplikationsfaktor von x;

o EXP = Exponentialfunktion zur Basis e = 2,71;

o SIN = Sinusfunktion.

k1 - k6 sind Parameter, die in gewissen Grenzen frei wählbar sind, um dem Amplitudenverlauf unterschiedliche Formen zu geben (vgl. Ausführungsbeispiele Fig.2 und Fig.3).

- x ist eine Bezugsgrösse welche eine Rechengrösse darstellt, die stellvertretend benutzt ist, um den zeitlichen Verlauf des Magnetfeldsignals innerhalb eines Hauptimpulses wiederzugeben. Während des Zeitintervalls zwischen dem Beginn t1 und dem Ende t2 eines Hauptimpulses durchläuft x die Werte von x1 bis x2, also x1 < x < x2. Damit ist jedem Zeitpunkt innerhalb eines Hauptimpulses eine Amplitude y zugeordnet. Die Grenzen für x1 liegen bei -5 < und für x2 bei +5.

Die wählbaren. Parameter k1 bis k6 haben folgende Bedeutung und Auswirkung auf das Magnet feldsignal:

- k1 ist ein voreinstellbarer Grundamplitudenwert mit dem sich ein Grundsignalwert bzw. ein Vormagnetisierungswert festlegen lässt, auf dem die Hauptimpulse 10 "aufsetzen" (Nulliniensymmetrie oder Nullinienasymmetrie). Dieser Grundwert muß keinem festgewählten Amplitudenwert entsprechen, sondern kann in der zeitlichen Abfolge für jeden Hauptimpulse individuell variieren (vgl. Kurvenverläufe in Fig. 3 a, b, c, d, e, f).

- k2 ist ein Multiplikationsfaktor für die EXP-Funktion; je grösser k2 gewählt wird, umso grösser wird der maximal erreichbare Modulations-Amplitudenwert eines Hauptimpulses, der sich zum Grundamplitudenwert addiert. k1 und k2 lassen sich - abhängig von k6 - derart begrenzen, dass die in einzelnen Ländern unterschiedlichen zulässigen Feldstärken nicht überschritten werden (vgl. Kurvenverläufe Fig. 3 d, e, f).

- k3 bestimmt als eine Art zeitlicher Skalierungsfaktor zusammen mit k4 und k5 den zeitlichen Verlauf (Steilheit und Amplitude) des modulierten Signal- bzw. Magnetisierungswerts der einzelnen Unterimpulse. Je größer der Parameter k3 gewählt wird, desto grösser die "Welligkeit" der die Extremalwerte der Unterimpulse verbindenden Hüllkurven (vgl. Kurvenverläufe Fig. 3 d und e).

- k4 bestimmt zusammen mit k3 und k5 ebenfalls Anzahl und Flankensteilheit insbesondere aber, als eine Art Dichtefaktor, die zeitliche Abfolge der einzelnen Unterimpulse der Unterimpulse, (k4≠0); Je größer der Parameter k4 gewählt wird, desto dichter sind die einzelnen Unterimpulse in den einzelnen "Wellen" der Hüllkurven jedes Hauptimpulses eingelagert (vgl. Kurvenverläufe Fig. 3 a, b, f).

- k5 ist, ähnlich wie k3, eine Art zeitlicher Skalierungs- und Dimensionierungsfaktor, über den sich In Verbindung mit den Parametern k3 und k4 der zeitliche Modulationsverlauf (Steilheit, Amplitude, und Dichte in der Abfolge innerhalb der oben beschriebenen "Wellen") des Signal- bzw. Magnetisierungswerts der einzelnen Unterimpulse innerhalb eines Hauptimpulses einstellen lässt. (vgl. Kurvenverläufe Fig. 3 c, d).

- k6 ist ein Normalisierungs- und Dimensionierungsfaktor zur Einstellung eines von k1 und k2 abhängigen und für die Abfolge der einzelnen Hauptimpulse festlegbaren mittleren Amplitudenwerts. Er gibt an mit welcher Steilheit die Amplitude des Hauptimpulses während der "aktiven" Impulsdauer zu- oder abnimmt. k6 beschreibt somit auch den mittleren Amplitudenzuwachs der in den Hauptimpuls eingelagerten Unterimpulse. Beispielsweise führen Werte k6 > 0 zu im Mittel zunehmenden bzw. bei k6 < 0 zu im Mittel abnehmenden - besonders durch die die Extremwerte der Unterimpulse verbindenden Hüllkurven verdeutlichten - Amplitudenwerten der Unterimpulse (vgl. Kurvenverläufe Fig. 3 a, b, c). Dies wirkt sich dies besonders auf die der Abszissenachse abgewandte Hüllkurve aus. Erfindungsgemäss bleibt hier insbesondere der Abstand der der Abszissenachse zugewandten dagegen nahezu konstant. Bei k6 = 0 bleiben die Amplitudenwerte der Unterimpulse im Mittel konstant und die Hüllkurven verlaufen parallel zur x-Achse.

Wie im Ausführungsbeispiel und in Fig. 2 bis Fig. 5 weiter ausgeführt, lässt sich auf diese Weise mittels geeignet gewählter Parametervariationen eine Abfolge von Hauptimpulsen mit - hinsichtlich mittlerer Grösse und Abstufungsfeinheit - individuell oder auch gruppenweise unterschiedlichen Unterimpulsen generieren.

Im wesentlichen Unterschied zu EP 0 995 463 B1 in welcher die Unterimpulse hinsichtlich ihrer Amplituden und Flankensteilheiten vom Beginn eines Hauptimpulses bis zum Ende eines Hauptimpulses stetig anwachsen und sich im Mittel weiter von der Koordinatenachse entfernen, entfällt in der vorliegenden Vorrichtung die durch die zeitliche Abfolge von Amplituden- und Steilheitsverhältnisse in EP 0 995 463 B1 formal vorgegebene Einschränkung der biologischen Aktivierungsmöglichkeiten.

Durch die feinere, am Beispiel der Pulsation der Hüllkurve besonders verdeutlichte Abstufung der Flankensteilheiten und Amplitudengrössen (letztlich auch durch die daran gekoppelten Dauer der einzelnen Unterimpulse), ist die spektrale Zusammensetzung jedes Hauptimpulses im Sinne der angestrebten Aufgabenlösung, eine möglichst breite Auswahl der an der Regulation beteiligten molekularen Steuerungsmechanismen anzusprechen, dementsprechend verbessert.

Die vorliegende Vorrichtung führt zu einer schnelleren und breiteren Anregung von den an den Regulationsmechanismen beteiligten molekularen Interaktionen und Stoffwechselvorgängen. Gestützt auf die oben ausgeführten Befunde könnte das darauf beruhen, dass die gegenüber EP 0 995 463 B1 wesentlich erhöhte Zahl speziell geformter (z.B. durch Amplitude und Flankensteilheiten charakterisierten) Unterimpulse für die zur Aufrechterhaltung der Regulationsvorgänge jeweils notwendigen individuellen energetischen Aktivierungen eine entsprechend breitere Auswahl an Energiemengen zur Verfügung stellt.

Bezogen auf die grundlegende Bedeutung dieser für den Lebensablauf entscheidenden Regulationsmechanismen lassen sich mit einer solchen Feldbeaufschlagung somit vorteilhaftere Wirkungen bei verschiedensten medizinischen Anwendungen erzielen. Beispielsweise bezogen auf die Prozesse zur Verbesserung des Funktionszustands der Mikrozirkulation und der damit erhöhten $O_2$-Utilisation führt dies zu einer weiter erhöhten Produktion des die Prozesse der Transkription, der Translation, der Formation und Modulation des Aktivitätszustands von Proteinen energetisch unterstützenden Energieträgers ATP, und - in weiterer Konsequenz auch zu einer beschleunigten Einstellung der Proteomik, also einer effizienteren Bereitstellung der diese Regulationen modulierenden Proteine.

Insbesondere kann dies auch zu einer weiter verbesserten Unterstützung der überaus komplex über Signalstoff- und unterschiedlichste Formen von Adhäsionsmolekülen gekennzeichneten Abfolge von Immunabwehrreaktionen führen.

In Verbindung mit einer erhöhten Syntheseleistung führt eine erhöhte $O_2$-Utilisation unter anderem auf der einen Seite zu einer verstärkten Bindegewebs- und Knorpelbildung und einer zusätzlichen Vaskularisation und verstärkt somit auch die von $O_2$-Diffusion im Knorpel stark abhängige Bildung von Chondrozyten. Durch eine formerhaltende und regenerationsfördernde Wirkung dieser elektrisch induzierten Knochenbildung gelingt es so dem Organismus mit einem Minimum an Material und Energie die nötigen Strukturen und verletzungs- und erkrankungsbedingte Störungen im Knochenaufbau beschleunigt zu beheben.

Auf der anderen Seite kann die bioelektrische Wirkung der induzierten Spannungen, in Verbindung mit einer Aktivierung der die ATP Bildung unterstützenden Protonenpumpen und des Weiteren begünstigt durch die $O_2$-Utilisation, über einen erhöhten Ionenaustausch zu einer Mineralisation des Bindegewebes führen. Wegen des eng an den Knochenstoffwechsel gekoppelten Auf- und Abbauprozessen von Knorpel kann die erfindungsgemässe Vorrichtung auch die für die Konsolidierung von Knochenfragmenten mitentscheidende Kalzium Ein- und Ausflußkinetik von Chondrozyten beeinflussen.

Die für intra- und interzellulären Signal- und Stoffvermittlung wichtigen Membranen der Membransysteme werden entweder direkt oder durch die im Kollagen gebildeten Potentiale beeinflußt oder auch über eine Änderung der Mikroumgebung der Zelle. Dieser Mechanismus basiert, möglicherweise ebenfalls wie oben in Verbindung mit einer elektromagnetischen Unterstützung der Protonentransportmechanismen, vermutlich auf einer elektrochemischen Übertragung, die die Zellaktivität durch Verschiebung der Ionenatmosphäre im extra- und damit auch im intrazellulären Raum modifiziert. Die kapazitive Aufladung der Zellmembran durch die elektrische Komponente der pulsierenden elektromagnetischen Felder stellt dabei einen entscheidenden Faktor dar. Verursacht durch die Struktur- und Ladungsverschiebung in der Membran, insbesondere im Bereich der Poren, besteht die Möglichkeit einer Permeabilitätsänderung mit einer daraus resultierenden Beeinflussung passiver Ionentransport- und Diffusionsvorgänge. Durch die enge Kopplung Oberflächenreaktion und Transmembrantransport scheinen neben der Protonenpumpe auch weitere membrangebundene Proteine, wie beispielsweise die Na-K-Pumpe, wichtige Empfängerstrukturen für die induzierte Energie darzustellen. Dabei kann eine gesteigerte Na-K-Adenosintriphosphatase-Aktivität eine verstärkte Natriumzufuhr durch die zuständige Ionenpumpe bewirken. Dabei führt nur die Anregung mit einem optimalen, erfindungsgemäßen Amplitudenverlauf der Hauptimpulse über vermutlich eine Erhöhung der Oberflächenkonzentration der entsprechenden Ionen zur Anregung der aktiven Transportkomplexe.

In seiner positiven Auswirkung auf die komplex vernetzten, vielschichtigen Regulationsprozesse im Lebensablauf könnte die Liste der vorteilhaft unterstützten Einzelmechanismen auf diese Weise weiter ausgedehnt werden. Abgesehen von der vorgenannten allgemeinen positiven Auswirkung, auch auf den Leistungssport, kann die erfindungsgemässe Vorrichtung somit ganz allgemein zur optimierten Unterstützung allgemeiner naturgegebener Schutz- Heil- und Erhaltungsprozesse und des Wohlbefindens beitragen.

In Verbindung mit der Reduktion des Auftretens allgemeiner chronischer Störungen könnte diese Erfindung über ihre die Beeinflussung lebenswichtiger Prozesse des Substanz- und Energieumsatzes und damit auch der Reduktion daran gekoppelter Medikationen letztlich auch die Entwicklung allgemeiner Krankheitskosten weiter eingrenzen.

Besonders vorteilhaft an der erfindungsgemäßen Vorrichtung ist, dass sie auch bei einer lokalen Behandlung zu einer Anregung der Stoffwechselvorgänge im gesamten Körper des Patienten führen kann.

Gute Ergebnisse lassen sich auch erreichen, wenn während einer Behandlungsdauer die Parameter der Amplitudenfunktion y nicht konstant gehalten werden, sondern nach einem auf den Patienten abgestimmten Muster variiert werden. Dazu werden Gruppen von Parameter definiert, die dann in zu wählenden Zeitperioden nacheinander zur Anwendung gelangen.

Konkretes Ausführungsbeispiel

Eine für die meisten Gewebearten generell sehr gute Stimulation lässt sich mit dieser Formel

$$y(x) = k1 + k2*EXP(SIN(x^{k3})+SIN((x*k4)^{k5})+x*k6);$$

erzielen wenn für den Verlauf eines Hauptimpulses die Funktionsparameter k1 bis k6, aus den folgenden Wertebereichen gewählt werden (detailliertere Funktionsbeschreibung zu siehe oben unter Aufgabe und Problemlösung):

$$-6 < k1, k2, k6 < 6 \text{ in (auch möglich in Schritten von 0,1)}$$

$$-6 < k3, k5 < 6 \text{ (ganzahlig)}$$

$$-10 < k4 < 10$$

(vergl. hierzu auch Fig. 2 bis Fig. 4)
Dies gilt mit

$$y(x) = EXP(SIN(x^3)+SIN((x*2)^3)+x*0,2);$$

beispielsweise für die folgenden Parameterwerte im Bereich von -5 < x < 5:

$$k1 = 0; k2 = 1; k3 = 3; k4 = 2; k5 = 3; k6 = 0,2;$$

Die obige Funktion gibt für jeden Hauptimpuls 10 den Verlauf der Amplitude y(x) und damit auch y(t) wieder, da t eine Funktion von x ist. Der Ablauf eines jeden Hauptimpulses 10 beginnt bei z.B. x = -5 und endet bei x = +5. Diese Formel gibt nur den Verlauf der "aktiven" Impulszeit an. Die jeweils vorzusehenden Ruhepausen zwischen den Hauptimpulsen /ta - tb/ werden durch diese Formel nicht beschrieben. In der Zeit zwischen den Hauptimpulsen, also den Ruhepausen nimmt das Signal bzw. das pulsierende Magnetfeld den Wert 0 oder einen voreinstellbaren festen Wert an. Der Bereich, den der Parameter x durchläuft, ist voreinstellbar und hängt weder vom Taktverhältnis noch von der Impulswiederholfrequenz ab. Mit anderen Worten ausgedrückt bedeutet das, dass jedem Hauptimpuls, gleichgültig von seiner Dauer ein Zeitverlauf zugeordnet ist, der durch die obige Formel bestimmt wird.

Anhand der Zeichnungen (Fig. 1 bis Fig. 5) wird die Erfindung näher erläutert. Es zeigen

Fig. 1 eine schematische Darstellung einer Ausführungsform der erfindungsgemäßen Vorrichtung zur Beeinflussung biologischer Abläufe;

Fig. 2 bis Fig.4 jeweils den prinzipiellen zeitlichen Verlauf der Hauptimpulse in für verschiedene Parameterwerte k1- k6 und dem Zeitabschnitt von x = -5 bis x = 5;

Fig. 5 einen zeitlichen Ausschnitt einer prinzipiell möglichen, durch Impulspausen 13 getrennten Abfolge von gemäss Fig. 2 bis Fig. 6 individuell gestalteten Hauptimpulsen in einem größeren zeitlichen Maßstab während einer Behandlungszeit 15. Durch unterschiedliche Parameterwerte gekennzeichnete Hauptimpulse sind hier zur Vereinfachung durch hinsichtlich Schraffur und Höhe unterschiedlich gekennzeichnete Vierecke symbolisiert. Je nach Vorgabe können sich darin unterschiedliche Hauptimpulse, auch gruppenweise 14, in ihrer zeitlichen Abfolge ablösen.

Im Einzelnen zeigt Fig. 1 eine erfindungsgemäße Vorrichtung bestehend aus zumindest einem Impulsgenerator 1, der in Spule 2 ein pulsierendes magnetisches Feld erzeugt, das in dem lebenden Gewebe 3, insbesondere im Körper eines zu behandelnden Patienten, zur Wirkung kommt. Zur Einstellung, insbesondere Optimierung, der Impulsparameter des pulsierenden Magnetfeldes im Generator 1 kann ein Sensor 4 bestimmte Körperparameter erfassen. Zu solchen Körperparametern zählen beispielsweise die Temperatur, der Blutdruck, die Pulsfrequenz oder der Sauerstoffgehalt des Blutes. Der erfaßte Parameter wird über eine Rückkopplungsleitung 5 einem Steuergerät 6 zugeführt, das den Parameter auswertet und den Impulsgenerator 1 entsprechend steuert. Für eine verbesserte Optimierung ist es möglich, mehrere Körperparameter zur Optimierung des pulsierenden Magnetfeldes gleichzeitig zu erfassen und auszuwerten. In Abhängigkeit von diesen Wirkungen kann das Steuergerät 6 jeweils die optimalen Werte für die Parameter k1 bis k6 auch automatisch festlegen.

Außerdem kann ein Sensor zur Erfassung der Frequenzabhängigkeit der auf den Körper übertragenen Wirkung der Felderzeugungsvorrichtung 2 vorgesehen sein. Aus den Unterschieden, insbesondere in der spektralen Zusammensetzung zwischen der von der Felderzeugung erzeugten Feldenergie und dem vom Sensor erfassten, ermittelt das Steuergerät den auf den behandelten Körper übertragenen Anteil.

In Abhängigkeit von dieser Wirkung legt das Steuergerät 6 die optimalen Werte für die Parameter k1 bis k6 selbst fest.

Bei solchen Felderzeugungsvorrichtungen 2 können die Feldstärken zusätzlich innerhalb der Geometrie der Felderzeugungsvorrichtung 2 variiert werden.

Mit dem Verfahren und der Vorrichtung der Erfindung wird ein pulsierendes Magnetfeld derart erzeugt, dass eine Abfolge von Hauptimpulsen 10 erzeugt wird, deren Form, abhängig von den Parametern k1, k2, k3, k4, k5, k6 prinzipiell den in Fig. 2 bis Fig. 4 dargestellten Verlaufsformen entspricht. Die Hauptimpulse 10, aus denen sich das gepulste Magnetfeld zusammensetzt beginnen zu einem Zeitpunkt t1 und erreichen, abhängig vom Vorzeichen von k6 ihren mittleren Mindest- oder Maximalwert. Die mittlere Amplitude des Hauptimpulses 10, bzw. die hierin periodisch modulierten Amplituden steigen (bei k6 > 0) bzw. fallen (k6 < 0) im Mittel im Ablauf jedes eines Hauptimpulses. Zu- bzw. Abnahme erfolgen gemäss einer Exponentialfunktion. Es sind aber auch andere Funktionen denkbar die den mittleren Anstieg (Abfall) der Amplitude eines Hauptimpulses innerhalb der Zeit beschreiben. Die optimale Form der Abfolge der Unterimpulse ist individuell sehr verschieden. Sie hängt ab von der Art des vom Feld beaufschlagten Gewebes, von dem gewünschten Heilerfolg und von dem jeweiligen Individuum ab.

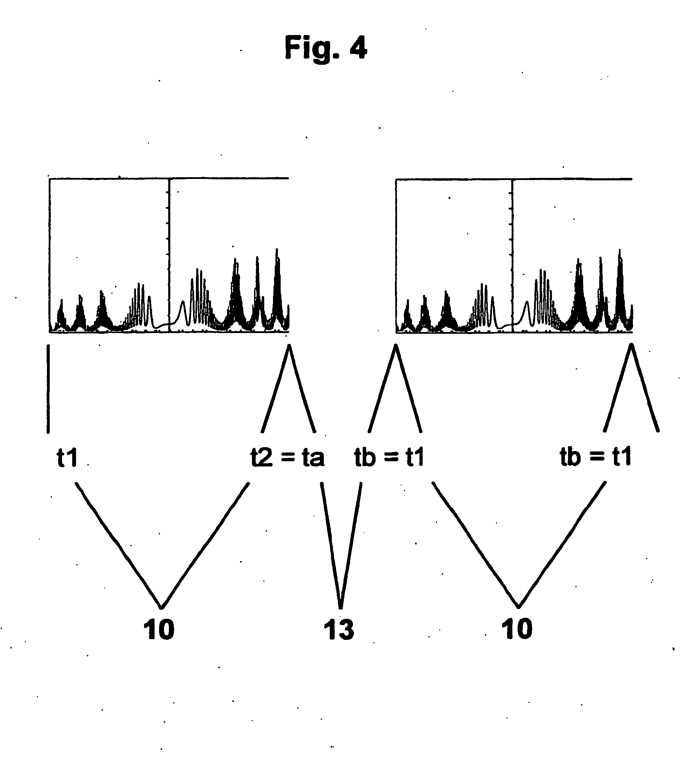

Die Abfolge einzelner Hauptimpulse kann durch Ruhepausen 13 getrennt sein vgl. Fig. 4. Es hat sich gezeigt, daß die optimale Grundfrequenz inklusiver dazwischenliegender wählbarer Pausen zwischen 0,01 und 1000 Hz schwanken kann. Diese Pausen sind vermutlich aufgrund der Relaxationszeit der Austauschprozesse erforderlich und führen erfahrungsgemäß zu einer besseren Anregung des lebenden Körpergewebes. Sie liegen beispielsweise in der Grössenordnung 0 bis 200 ms. Das Tastverhältnis zwischen Ruhezeit (Zeitpunkte ta bis tb in Fig. 4) und Impulswiederholdauer T liegt vorzugsweise zwischen 0% und 300%.

Jeder Hauptimpuls 10 ist mit Unterimpulsen 11 moduliert. Zu Beginn jedes Hauptimpulses 10 bei t1 beginnt die Amplitude der überlagerten Unterimpulse 11 mit einem zuvor gewählten Magnetisienrngswert und steigt bzw. fällt abhängig vom Vorzeichen von k6 bis zum Ende des Hauptimpulses zum Zeitpunkt t2. Zwischen dem Zeitpunkt t1 und dem Zeitpunkt t2 (Dauer eines Hauptimpulses 10) verändert sich zwar die Amplitude der überlagerten Unterimpulse, unterliegt aber hinsichtlich der zeitlichen Abfolge innerhalb eines Hauptimpulses (wesentlich abhängig von den Parameterwerten k3; k4, und k5) unterschiedlich dicht aufeinander folgenden Pulsationen. Der mittlere Abstand zwischen der x-Koordinatenachse und einer ihr zugewandten Hüllkurve kann, abhängig von k6, jedoch weitgehend konstant gehalten werden.

Insbesondere zeigen die Kurvenverlaufe in Fig.2 und Fig. 3, dass die Dichte und damit auch Steilheit der im Hauptimpuls 10 aufeinanderfolgender Unterimpulse 11 und somit auch die "Welligkeit" (Pulsationen) der ihre Extrema verbindenden Hüllkurve 12, abhängig von den jeweiligen x-Werten symmetrisch zur Ordinatenachse bei x = 0 mit zunehmenden Betragswerten von x und zunehmenden Werten von k3, k4, und k5 ständig steigt (vgl. insbesondere Kurvenverläufe Fig. 3 d, e, f).

Im Zeitablauf eines Hauptimpulses, also zwischen den Zeitpunkten t1 und t2 verändern sich wesentlich abhängig vom Parameterwert k6 desweiteren auch die Amplituden dieser nach obiger Formel erfindungsgemäss modulierten Unterimpulse. Die mittleren Amplituden dieser Unterimpulse 11, bzw. der mittlere Abstand der ihre Extrema verbindenden Hüllkurven 12 von der x-Achse, nehmen für Werte von k6 > 0 im Mittel bei sonst gleichen Werten für k1 bis k5 kontinuierlich zu bzw. für Werte k6 < 0 ständig ab. Für k6 = 0 bleiben die Amplituden und dementsprechend auch der mittlere Abstand zwischen Hüllkurven und der x-Achse konstant. (Repräsentativ für k6 sind in Fig. 3 nur Kurvenverläufe für k6 > 0 dargestellt, die Kurvenverläufe für negative Werte von k6 entstehen durch Spiegelung an der x = 0 Ordinate).

Diese überlagerten Unterimpulse 11 führen zur Stimulation der physiologischen Austauschprozesse und tragen damit entscheidend zur Beschleunigung der angesprochenen Regulations- und Heilungsvorgänge bei. Wichtig ist dabei insbesondere, daß die Amplitude dieser Unterimpulse 11 im Verlauf eines jeden Hauptimpulses 10 variiert und, im wesentlichen Unterschied zu EP 0 995 463 B1, dass diese Unterimpulse hinsichtlich Flankensteilheit Amplitude und Zeitablauf wesentlich feiner abgestuft sind.

Die optimale Form und Abfolge der Unterimpulse ist individuell sehr verschieden. Sie hängt ab von der Art des vom Feld beaufschlagten Gewebes, von dem erwünschten Heilungserfolg und vom jeweiligen Individuum.

Eine entscheidende Bedeutung bei der Stimulation der Austauschprozesse im Körpergewebe hat vermutlich der hohe, durch die Vielzahl der überlagerten Unterimpulse 11 bedingte Anteil der an- bzw. absteigenden Flankenabschnitte.

Wenn mit Hilfe von Sensoren bestimmte Parameter des lebenden Gewebes, insbesondere des menschlichen Körpers, erfasst werden, lässt sich der Verlauf jedes Hauptimpulses 10 derart an die tatsächlichen Verhältnisse anpassen, dass eine optimale Stimulation erreicht wird. Dazu werden in Abhängigkeit von den erfaßten Gewebeparametern

Eine Optimierung der Wirkung der vorliegenden Vorrichtung auf den Organismus kann durch eine Rückkopplung verbessert werden. Zu diesem Zweck können Sensoren verwendet werden, die einen oder mehrere verschiedener Körperparameter messen, um die Anregung des Körpers durch die elektromagnetischen Pulse zu optimieren. Mit den Sensoren lassen sich Gewebeparameter wie beispielsweise Blutdruck, Temperatur, Puls, ph-Wert oder Atemvolumen erfassen und im Sinne einer adaptive Anpassung der Stimulation an die Sensibilität des zu stimulierenden Gewebes zur Optimierung der Parameter der Vorrichtung zur Erzeugung elektromagnetischer Felder verwenden. Insbesondere liessen sich über eine derartige Rückkopplungsschleife eventuelle durch die Anregung selbst verursachte Sensibilitätsänderungen im beaufschlagten Gewebe ausgleichen.

Eine Optimierung ergibt sich desweiteren aus der Erfassung der Frequenzabhängigkeit der auf den Körper übertragenen Wirkung. Aus den Unterschieden, insbesondere in der spektralen Zusammensetzung zwischen den von der Felderzeugung 2 erzeugten Feldenergie und der vom Sensor erfassten, ermittelt das Steuergerät den auf den behandelten Körper übertragenen Energieanteil. In Abhängigkeit davon könnte das Steuergerät 6 die optimalen Werte für die Parameter k1 bis k6 selbst festlegen.

## Patentansprüche

1. Vorrichtung zur Beeinflussung biologischer Abläufe in einem lebenden Gewebe, insbesondere einem menschlichen Körper, zur Beaufschlagung zumindest eines Teils des Gewebes mit einem pulsierenden magnetischem Feld, mit einer Felderzeugungsvorrichtung (2) zur Erzeugung des pulsierenden magnetischen Feldes und einem Impulsgenerator (1) zur Ansteuerung der Felderzeugungsvorrichtung (2),
   **dadurch gekennzeichnet,**
   **dass** der Impulsgenerator (1) derart ausgebildet ist, dass das pulsierende Magnetfeld aus einer Folge von Hauptimpulsen (10) besteht deren Impulswiederholungsrate zwischen 0,01 und 1000 Hz liegt,
   **dass** der Amplitudenverlauf eines Hauptimpulses die folgende Funktion aufweist:

$$y(x) = k1 + k2*EXP(SIN(x^{k3})+SIN((x*k4)^{k5})+x*k6);$$

   darin bedeuten:

   $y(x)$ = Amplitude des Magnetfeldes innerhalb eines Hauptimpulses als Funktion von x;
   x = rechnerische Ersatzgrösse für die Zeit t während eines Hauptimpulses;
   k1 = Zahlenwert;
   k2 = Multiplikationsfaktor für die Funktion $EXP(SIN(x^{k3})+SIN((x*k4)^{k5})+x*k6)$;
   k3 = Exponent von x;
   k4 = Multiplikationsfaktor von x, k4≠0;
   k5 = Exponent von (x*k4);
   k6 = Multiplikationsfaktor von x;
   EXP = Exponentialfunktion zur Basis e = 2,71;
   SIN = Sinusfunktion.

   k1 - k6 sind Parameter, die in gewissen Grenzen frei wählbar sind, um dem Amplitudenverlauf unterschiedliche Formen zu geben, wobei jeder Hauptimpuls durch entsprechende Wahl der Parameter mit Unterimpulsen (11) moduliert ist und die rechnerische Ersatzgrösse x für die Zeit t von -5<x<+5 variiert.

2. Vorrichtung nach Anspruch 1, **dadurch gekennzeichnet, dass** die Parameter der Funktion mit den Eigenschaften k1 bis k5 ganzzahlig und k6 in dezimaler Abstufung aus den folgenden Wertebereichen gewählt werden:

- 6 < k1, k2, k6 < 6 in (auch möglich in Schritten von 0,1)
- 6 < k3, k5 < 6 (ganzzahlig)
- 10<k4<10,

beispielsweise: k1 = 0; k2 = 1; k3 = 3; k4 = 2; k5 = 3; k6 = 0.2.

**3.** Vorrichtung nach Anspruch 1 oder 2,
**dadurch gekennzeichnet, dass** aufeinanderfolgende Hauptimpulse mit unterschiedlichen Kombinationen von Parameterwerten einstellbar und auswählbar sind.

**4.** Vorrichtung nach Anspruch 3,
**dadurch gekennzeichnet, dass** eine Gruppe von unterschiedlichen Kombinationen von Parameterwerten (14) abrufbar ist, um sie in definierter zeitlicher Abfolge zur Anwendung zu bringen, wobei die Dauer einer Impulsgruppe (14) zwischen 0,25 Sekunden und 1,2 Sekunden liegt.

**5.** Vorrichtung nach Anspruch 4, **dadurch gekennzeichnet,
dass** die Dauer der Impulsgruppen (14) während der Dauer der Beaufschlagung des Gewebes mit dem pulsierenden Magnetfeld in Abhängigkeit von der Zeit variabel gestaltet ist.

**6.** Vorrichtung nach wenigstens einem der Ansprüche 1 - 5, **dadurch gekennzeichnet,
dass** das Verhältnis zwischen den Hauptimpulsen (10) und den dazwischen liegenden Ruhepausen innerhalb der Impulsgruppen (14) zwischen 0% und 300% beträgt.

**7.** Vorrichtung nach wenigstens einem der Ansprüche 1 bis 5, **dadurch gekennzeichnet, dass** die Vorrichtung außerdem umfaßt:

mindestens einen Sensor (4) zur Erfassung jeweils eines Gewebeparameters und ein Steuergerät (6) zur Auswertung des Gewebeparameters, dem der von dem mindestens einen Sensor (4) erfaßte Gewebeparameter zugeführt wird

**8.** Vorrichtung nach wenigstens einem der Ansprüche 1 bis 5, **dadurch gekennzeichnet, dass** die Vorrichtung außerdem umfaßt

mindestens einen Sensor (4) zur Erfassung der Differenz zwischen der applizierten und der vom Körper aufgenommenen Feldenergie und
ein Steuergerät (6), dem der von dem mindestens einen Sensor (4) erfaßte Energiewert zugeführt wird.

**Claims**

**1.** Device for influencing biological processes in living tissue, particularly a human body, for exposing at least a portion of the tissue to a pulsating magnetic field, with a field generating device (2) for generating the pulsating magnetic field and a pulse generator (1) for controlling the field generating device (2),
**characterized in**
**that** the pulse generator (1) is designed such that the pulsating magnetic field is composed of a sequence of main pulses (10) having a pulse repetition rate between 0.01 and 1000 Hz,
**that** the amplitude response of a main pulse has the following function:

$$y(x) = k1 + k2*EXP(SIN(x^{\wedge}k3)+SIN((x*k4)^{\wedge}k5)+x*k6);$$

where:

y(x) = amplitude of the magnetic field within a main pulse as a function of x;

x = mathematical substitute measure for the time t during a main pulse;
k1 = digit;
k2 = multiplication factor for the function EXP(SIN(x^k3)+SIN((x*k4)^k5)+x*k6);
k3 = exponent of x;
k4 = multiplication factor of x, k4≠0;
k5 = exponent of (x*k4);
k6 = multiplication factor of x;
EXP = exponential function to base e = 2,71;
SIN = sine function.

k1 - k6 are parameters that are freely selectable within certain limits, in order to give the amplitude response different forms, wherein each of the main pulses is modulated with sub-pulses (11) by respective choice of the parameters, and wherein the mathematical substitute measure x for time t varies from -5 <x <+5.

2. Device according to claim 1, **characterized in that** the parameters of the function with the properties k1 to k5 being integers and with k6 having a decimal gradation are selected from the subsequent ranges of values:

- 6 < k1, k2, k6 < 6 in (also possible in steps of 0,1)
- 6 < k3, k5 < 6 (integer)
- 10 <k4< 10,

for example: k1 = 0; k2 = 1; k3 = 3; k4 = 2; k5 = 3; k6 = 0,2.

3. Device according to claim 1 or 2,
**characterized in that** successive main pulses are adjustable and selectable with different combinations of parameter values.

4. Device according to claim 3,
**characterized in that** a group of different combinations of parameter values (14) is retrievable, in order to apply it in defined time sequence, wherein the duration of a pulse group (14) is between 0,25 seconds and 1.2 seconds.

5. Device according to claim 4,
**characterized in that** the duration of the pulse groups (14) during the durations of exposing the tissue to the pulsating magnetic field is made variable depending on the time.

6. Device according to at least one of claims 1 to 5,
**characterized in that** the ratio between the main pulses (10) and the intervening rest periods within the pulse groups (14) is between 0% and 300%.

7. Device according to at least one of claims 1 to 5,
**characterized in that** the device further incudes:

at least one sensor (4) for determining a respective tissue parameter, and a control device (6), which is supplied with the tissue parameter determined by the at least one sensor (4), for evaluating the tissue parameter.

8. Device according to at least one of the claims 1 to 5,
**characterized in that** the device further incudes:

at least one sensor (4) for detection of the difference between the applied field energy and the field energy absorbed by the body, and a control device (6), which is supplied with the energy value detected by the at least one sensor (4).

**Revendications**

1. Dispositif pour influer sur les processus biologiques dans les tissus vivants, en particulier un corps humain, pour exposer au moins une partie du tissu à un champ magnétique pulsé, d'un dispositif de génération de champ (2) pour générer le champ magnétique pulsé et un générateur d'impulsions (1) pour commander le dispositif de génération de champ (2),
**caractérisé en**
**que** le générateur d'impulsions (1) est conçu de telle sorte que le champ magnétique pulsé est composé d'une séquence d'impulsions principal (10) ayant un taux de répétition des impulsions entre 0,01 et 1000 Hz,
que la réponse en amplitude d'une impulsion principale a la fonction suivante:

$$y(x) = k1 + k2*EXP(SIN(x^k3)+SIN((x*k4)^k5)+x*k6);$$

où:

y (x) = amplitude du champ magnétique dans une impulsion principale en fonction de x;
x = mesure de substitution mathématique pour le temps t pendant une impulsion principale;
k1 = chiffres;
k2 = facteur de multiplication de la fonction EXP(SIN(x^k3)+SIN((x*k4)^k5)+x*k6);
k3 = exposant de x;
k4 = facteur de multiplication de x, k4 ≠ 0;
k5 = exposant de (x * k4);
k6 = facteur de multiplication de x;
EXP = fonction exponentielle de base e = 2,71;
SIN = fonction sinus.

k1 - k6 sont des paramètres qui peuvent être choisis librement dans certaines limites, afin de donner la réponse en amplitude différentes formes, dans laquelle chacune des impulsions principale est modulée avec des sous-impulsions (11) par choix respectifs des paramètres, et dans laquelle le substitut mathématiques mesure x au temps t varie de -5 <x <5.

2. Dispositif selon la revendication 1,
**caractérisé en ce que** les paramètres de la fonction avec les propriétés k1 à k5 étant des nombres entiers et k6 avoir une gradation décimales sont choisis parmi les gammes suivantes de valeurs:

- 6 <k1, k2, k6 <6 po (également possible en étapes de 0,1)
- 6 <k3, k5 <6 (entier)
- 10 <k4 <10,

par exemple: k1 = 0; k2 = 1; k3 = 3; k4 = 2; k5 = 3; k6 = 0,2.

3. Dispositif selon la revendication 1 ou 2,
**caractérisé en ce que** des impulsions successives principaux sont réglables et sélectionnables avec différentes combinaisons de valeurs des paramètres.

4. Dispositif selon la revendication 3,
**caractérisé en ce que** un groupe de différentes combinaisons de valeurs de paramètres (14) est récupérable, en vue de l'appliquer dans l'ordre de temps définie, dans laquelle la durée d'un groupe d'impulsion (14) est comprise entre 0,25 secondes et 1,2 secondes.

5. Dispositif selon la revendication 4,
**caractérisé en ce que** la durée de l'impulsion de groupes (14) pendant la durée de l'exposition du tissu à la champ magnétique pulsé est variable en fonction du temps.

6. Dispositif selon au moins une des revendications 1 à 5,
**caractérisé en ce que** le rapport entre les impulsions principal (10) et les périodes de repos intervient dans les groupes d'impulsions (14) est comprise entre 0% et 300%.

7. Dispositif selon au moins une des revendications 1 à 5,
**caractérisé en ce que** le dispositif en outre comprends:

au moins un capteur (4) pour déterminer un paramètre tissu respectif, et un dispositif de commande (6), qui est fourni avec le paramètre tissus déterminée par le capteur d'au moins un (4), pour évaluer le paramètre tissus.

8. Dispositif selon au moins l'une des revendications 1 à 5,
   **caractérisé en ce que** le dispositif en outre comprends:

   au moins un capteur (4) pour la détection de la différence entre l'énergie du champ d'application et l'énergie du champ absorbée par le corps, et un dispositif de commande (6), qui est fourni avec la valeur de l'énergie détectée par le capteur d'au moins un (4).

# Fig. 1

# Fig. 2

X= - 5          X = 0          x = +5

t1                              t2

# Fig. 3

a   y=60+1.5*exp(sin(x^3)+sin((x*4)^3)+x*0.3)

b   y=40+0.3*exp(sin(x^3)+sin((x*2)^3)+x*0.5)

c   y=27+1*exp(sin(x^3)+sin((x*2)^5)+x*0.1)

d   y=15+1*exp(sin(x^3)+sin((x*2)^3)+x*0.1)

e   y=10+0.3*exp(sin(x^2)+sin((x*2)^3)+x*0.1)

f   y=0+0.7*exp(sin(x^3)+sin((x*1)^3)+x*0.1)

# Fig. 4

t1    t2 = ta    tb = t1    tb = t1

**10**    **13**    **10**

# Fig. 5

15

13  10  13

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0995463 B1
- EP 0594655 B1, König Herbert
- EP 0729318 B1, Fischer Gerhard
- EP 0377284 A
- EP 1364679 A2
- EP 0266807 A
- EP 0266907 A
- DE 4221739 A
- US 5181902 A
- WO 9632159 A
- UA 4428366 A

**In der Beschreibung aufgeführte Nicht-Patentliteratur**

- Red blood metabolism and haemoglobin oxygen affinity: effect of electromagnetic fields on healthy adults. **SPODARYK K.** 2nd Int World Congress Bio-Electro-Magnetic-Energy-Regulation. Emphyspace. 2001, vol. 2, 15-19
- **KAFKA WA ; SPODARYK K.** Effects of extremely weak BEMER 3000 type pulsed electromagnetic fields on red blood metabolism and hemoglobin oxygen affinity. *Fizoterapia,* 2003, vol. 11 (3), 24-31
- **KLOPP R.** Vitalmikroskopische und reflexions-spektrometrische Untersuchungen zur Wirkung des Gerätesystems. *BEMER 3000'' auf den Funktionszustand der Mikrozirkulation,* 2004
- **KLOPP R ; NIEMER W.** Einfluss eines pulsierenden elektromagnetischen Feldes mit vasomotorischer Stimulation auf einen eingeschränkten Funktionszustand der Mikrozirkulation. Komplement. *Integr. Med,* August 2007, 47-53
- The electromagnetic BEMER 3000 signal modifies response to teratogens. **JELÍNEK R ; BLÁHA J ; DBAL**ý **JAROSLAV.** 3nd Int. World Congress Bio-Electro-Magnetic Energy-Regulation. 2002
- **KAFKA WA ; PREIßINGER M.** Verbesserte Wundheilung durch gekoppelte, BEMER 3000 typisch gepulste, Elektromagnetfeld- und LED-Licht-Therapie am Beispiel vergleichender Untersuchungen an standardisierten Wunden nach Ovariektomie bei Katzen (felidae. *Österreichische Gesellschaft der Tierärzte (ÖGT) Kleintiertage-Dermatologie,* 02. Marz 2002
- **SPODARYK K.** The effect of extremely weak electromagnetic field treatments upon signs and symptoms of delayed onset of muscle soreness: A placebo controlled clinical double blind study. *Medicina Sportiva,* 2002, vol. 6, 19-25
- **KLOPP R ; NIEMER W ; POMRENKE P ; SCHULZ J.** Magnetfeldtherapie: Komplementär-therapeutisch sinnvoll oder Unsinn?. *Stellungnahme unter Berücksichtigung neuer Forschungsergebnisse mit dem Gerätesystem BEMER 3000,* 2005
- The influence of extremely weak BEMER3000 typed pulsed electromagnetic fields on ratings of perceived exertion at ventilatory threshold. **SPODARYK K ; KAFKA WA.** Rehabilitation Sciences in the New Millennium Challenge for Multidisciplinary Research. 8th Congress of EFRR. 2004, 279-283
- **RIHOVA B.** Die Wirkung der elektromagnetischen Felder des BEMER 3000 auf das Wachstum des experimentellen Mäuse-EL 4T Zellen-Lymphoms. *SAMET Kongress,* 2004
- **KAFKA WA ; SCHÜTZE N ; WALTHER M.** Einsatz extrem niederfrequent (BEMER typisch) gepulster schwacher elektromagnetischer Felder im Bereich der Orthopädie (Application of extreme low frequent (BEMER type) pulsed electromagnetic fields in orthopedics. *Orthopädische Praxis,* 2005, vol. 41 (1), 22-24
- **WALTHER M ; MEYER F ; KAFKA WA ; SCHÜTZE N.** Effects of weak, low frequency pulsed electromagnetic fields (BEMER type) on gene expression of human mesenchymal stem cells and chondrocytes: an in vitro study. *Electromagnetic Biology and Medicine,* 2007, 257936
- **MICHELS-WAKILI S ; KAFKA WA.** BEMER 3000 pulsed low-energy electromagnetic fields reduce dental anxiety: a randomized placebo controlled single-blind study. *10th International Congress on Modern Pain Control,* 05. Juni 2003

- **BERNATZKY G ; KULLICH W ; AGLAS F ; AUSSERWINKLER M ; LIKAR R ; PIPAM W, H. ; SCHWANN H ; KAFKA WA.** Auswirkungen von speziellen, (BEMER-typisch) gepulsten elektro-magnetischen Feldern auf Schlafqualität und chronischen Kreuzschmerz des Stütz- und Bewegungsapparates (low back pain. *Eine doppelblinde randomisierte Duo Center Studie,* 2007
- **GABRYS M.** Pulsierende Magnetfeldtherapie bei zytostatisch bedingter Polyneuropathie. *Deutsche Zeitschrift für Onkologie,* 2004, vol. 36, 154-156
- Biological effects of electric and magnetic elds: sources and mechanism. **CARPENTER DO ; ARYAPETYAN S.** Biological effects of electric and magnetic elds: sources and mechanism. Academic Press, 1994, vol. 1
- Beneficial and harmful effects. Academic Press, vol. 2

- **BOHN W ; KAFKA WA.** Energie und Gesundheit: BEMER 3000 Bio-Elektro-Magnetische-Energie-Regulation. Haug Verlag, 2004, 1-130
- **KAFKA WA.** The BEMER 3000 Therapy: A new complementary ''electro-magnetic drug'' effectively supports widespread scattered prophylactic and therapeutic treatments. *Achievements in space medicine into health care practice and industry 3rd European praxis matured congress KOPIE-DRUCK,* 2006
- **QUITTAN M ; SCHUHFRIED O ; WIESINGER GF ; FIALKA-MOSER V.** Klinische Wirksamkeiten der Magnetfeldtherapie - eine Literaturübersicht. *Acta Medica Austriaca,* 2000, vol. 3, 61-68
- **MATTHES RUDIGER.** Guidance on determining compliances of exposure to pulsed and complex non sinusoidal waveforms below 100 khz with ICNIRP GUIDELINES. *The International Commission on Non-Ionizing Radiation Protection ICNIRP Secretariat, Bundesamt für Strahlenschutz,* 2003